# DEMANDE DE BREVET EUROPEEN

(11) **EP 1 190 686 A1**
(43) Date de publication de la demande: **27.03.2002**
(21) Numéro de dépôt: 01420198.2
(22) Date de dépôt: 19.09.2001
(51) Int. Cl.: A61F 2/38

(54) **Prothèse de genou, totale et modulaire**

(30) Priorité: 22.09.2000 FR 0012323
(71) Demandeur: Implants Industrie, 42480 La Fouillouse (FR)
(72) Inventeur: Raux, Philippe, 63122 Manson (FR); Buscayret, Christian, 34130 Mauguio (FR); Dufour, Guillaume, 91800 Brunoy (FR); Nathan, Jean-Louis, 75017 Paris (FR); Besse, Jean-Pierre, 27400 Couviers (FR); Granier, Eric, 34500 Beziers (FR); Paran, Michel, 34500 Beziers (FR)
(74) Mandataire: Perrier, Jean-Pierre

(57) **Abrégé**

Cette prothèse de genou comprend une embase tibiale (2), un élément condylien, avec un tenon axial enfichable dans la cavité médullaire fémorale, et un insert intermédiaire (4).

Selon l'invention, de la face inférieure du plateau de l'insert débouche un logement en forme de haricot, tandis que le plateau tibial comporte, en vis à vis du logement de l'insert, un autre logement en forme de haricot, en saillie de son fond, un doigt cylindrique et vertical, et que, dans les configurations de la prothèse où l'insert est calé en rotation par rapport au plateau tibial, la position angulaire de l'insert par rapport à ce plateau tibial est définie par une cale en forme de haricot, insérée pour partie dans ce logement et pour partie dans celui du plateau tibial, ladite cale étant munie d'un alésage coiffant le doigt saillant du fond du logement du plateau tibial étant choisie dans une série de plusieurs cales se différenciant par la position de l'axe de leur alésage.

## Description

L'invention est relative à une prothèse de genou, totale et modulaire.

Lors d'une d'intervention sur le genou et malgré un examen attentif des radiographies préopératoires, le chirurgien ne connaît pas totalement l'état du genou déficient et rencontre bien souvent des difficultés pour adapter parfaitement une prothèse à la morphologie du patient et à la pathologie rencontrée. Ces difficultés consistent par exemple à devoir remplacer la prothèse standard, envisagée initialement, par une prothèse de reprise nécessitant un ancillaire différent, et non préparé.

La présente invention a pour objet de fournir une prothèse de genou, totale et modulaire, remédiant à ces inconvénients et permettant avec les mêmes éléments de base, à savoir embase tibiale, insert tibial et élément condylien, combinés avec des éléments complémentaires, nouveaux ou connus, de satisfaire à toutes les pathologies avec le même ancillaire.

De façon connue, cette prothèse de genou comprend :
- une embase tibiale avec plateau solidaire d'un tenon axial enfichable dans la cavité médullaire tibiale,
- un élément condylien avec un tenon axial enfichable dans la cavité médullaire fémorale,
- un insert intermédiaire avec un plateau venant en appui sur le plateau tibial et présentant, de l'autre côté, des surfaces de roulement pour les condyles de l'élément condylien, et un éperon saillant vers le haut, cet éperon étant traversé par un alésage axial, coaxial à l'axe longitudinal du tenon de l'élément tibial,
- et des moyens complémentaires ménagés sur le plateau tibial et l'insert pour limiter leur rotation relative autour de l'axe longitudinal précité.

Selon l'invention, de la face inférieure du plateau de l'insert tibial débouche un logement en forme de haricot dont les rayons des bords en arc de cercle, respectivement antérieur et postérieur, sont centrés sur l'axe longitudinal de l'alésage axial et dont les arrondis extrêmes sont disposés symétriquement par rapport au plan antéro-postérieur de l'insert, tandis que le plateau tibial comporte, en vis à vis du logement de l'insert, un autre logement en forme de haricot, de même largeur mais s'étendant symétriquement sur un plus grand arc, ce logement comportant, en saillie de son fond, un doigt cylindrique et vertical, et que, dans les configurations de la prothèse où l'insert est calé en rotation par rapport au plateau tibial, la position angulaire de l'insert par rapport à ce plateau tibial est définie par une cale en forme de haricot, de même forme et dimensions que celles du logement de l'insert, insérée pour partie dans ce logement et pour partie dans celui du plateau tibial, ladite cale étant munie d'un alésage coiffant le doigt saillant du fond du logement du plateau tibial et assurant son calage par rapport à ce plateau et étant choisie dans une série de plusieurs cales se différenciant par la position de l'axe de leur alésage par rapport à leur plan médian transversal.

Avec cette prothèse, le chirurgien peut, durant l'intervention, choisir la version correspondante à la pathologie du patient, à savoir :
- avec inclusion d'une cale symétrique dans les logements en forme de haricot, obtention d'une prothèse standard postéro-stabilisée de première intention avec insert fixe sur l'embase tibiale,
- avec inclusion d'une cale en forme de haricot asymétrique, réalisation d'une prothèse avec insert tibial postéro-stabilisée, fixe mais décalé angulairement sur l'embase tibiale,
- sans inclusion de la cale, réalisation d'une prothèse avec insert tibial postéro-stabilisée, mobile en rotation sur l'embase tibiale,
- et avec des éléments complémentaires tels que rallonge tibiale, rallonge fémorale et cale de rattrapage sous le plateau de l'embase tibiale, obtention de prothèses de reprise adaptables dans les trois versions ci-dessus.

Il apparaît que, par un assemblage simple des composants de la prothèse, le chirurgien peut l'adapter à la morphologie et à la pathologie du patient, tout en utilisant une instrumentation et un ancillaire de pose, commun à toutes les versions.

A ces avantages, il faut ajouter une réduction des coûts de fabrication et de stockage, par utilisation des mêmes composants principaux, conduisant par l'augmentation des quantités de chaque composant, à une réduction du prix de revient et par la diminution du nombre de références devant être gérées aussi bien en fabrication que dans les stocks intermédiaires, à une réduction des frais de gestion et de stockage.

Dans une forme d'exécution de l'invention, le logement en forme de haricot de l'insert débouche d'une excroissance saillant de la face d'appui de cet insert, cette excroissance étant délimitée, vers l'arrière, par une face circulaire dont le rayon R1 est centré sur l'axe longitudinal de l'insert et vers l'avant par une face circulaire de grand rayon R2, cette excroissance étant engagée dans un logement ménagé dans le plateau tibial et délimité, vers l'arrière, par une face circulaire de même rayon R1 centré sur l'axe longitudinal du tenon tibial, et vers l'avant par une face circulaire de plus grand rayon R3 que celui R2 de la face correspondante de l'excroissance, de manière que, en configuration sans cale, donc avec rotation libre de l'insert, les extrémités des deux faces circulaires de grand rayon constituent butées de limitation de course angulaire.

D'autres caractéristiques et avantages ressortiront de la description qui suit en référence au dessin schématique annexé représentant, à titre d'exemple, une forme d'exécution de la prothèse, totale et modulaire, selon l'invention.

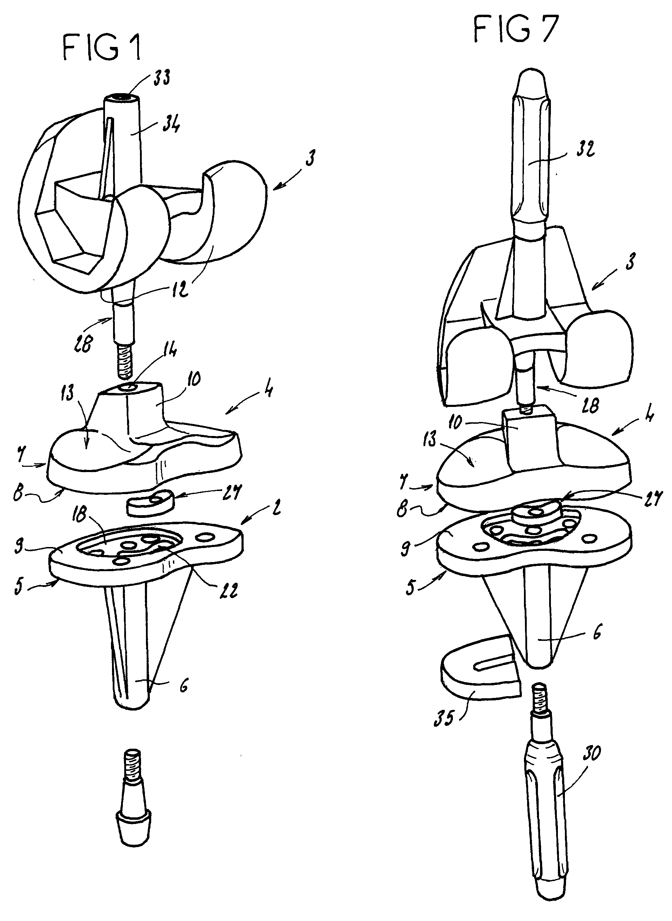

Figure 1 est une vue en perspective éclatée montrant les composants de la prothèse, lorsqu'elle est en configuration de prothèse standard postéro-stabilisée avec insert calé sur l'embase tibiale,
Figure 2 est une vue partielle en perspective éclatée des moyens de calage en rotation de l'insert par rapport au plateau tibial,
Figure 3 est une vue partielle, en coupe antéro-postérieure, montrant les moyens de liaison et de calage en rotation de l'insert avec le plateau tibial,
Figure 4 en est une vue en coupe suivant IV-IV de figure 3,
Figure 5 est une vue similaire à la figure 4, mais avec utilisation d'une cale asymétrique,
Figure 6 est une vue en coupe, similaire à la figure 3, mais lorsque la prothèse est sans cale, et permet la rotation de l'insert sur le plateau tibial,
Figure 7 est une vue en perspective de la prothèse, lorsqu'elle est en configuration de reprise.

De façon connue, la prothèse est composée d'une embase tibiale 2, d'un élément condylien 3 et d'un insert intermédiaire 4. L'embase tibiale 2 comprend un plateau 5 et un tenon axial 6 enfichable dans la cavité médullaire fémorale. L'insert 4 est également composé d'un plateau 7 dont la face d'appui 8 est apte à venir reposer sur la face d'appui 9 du plateau tibial 5. Le plateau 8 est solidaire d'un éperon 10 s'insérant entre les deux condyles 12 de l'élément condylien qui roulent sur des surfaces concaves 13 du plateau 8.

L'éperon 10 et le plateau 7 de l'insert 4 sont traversés verticalement par un alésage 14 dont l'axe longitudinal est coaxial avec l'axe longitudinal du tenon 6 de l'embase tibiale 2.

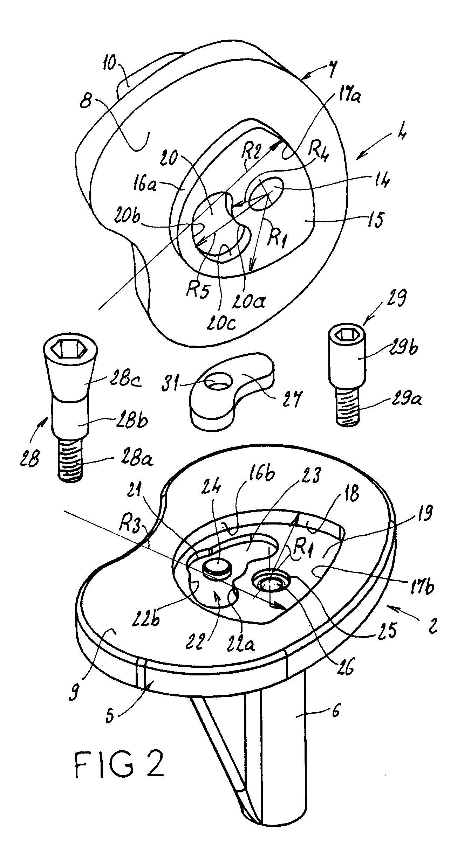

Selon l'invention, et comme montré à la figure 2, de la face 8 de l'insert 4 fait saillie une excroissance 15 délimitée vers l'arrière par une face circulaire 16a, de rayon R1 centré sur l'axe de l'alésage 14, et une face circulaire antérieure 17a, de rayon R2, plus grand que R1. Cette excroissance correspond à un logement 18, de forme similaire, ménagé dans le plateau 5 de l'embase tibiale 2 et débouchant de la face 9 de ce plateau. Ce logement est délimité entre une face arrière 16b, de rayon R1 centré sur l'axe longitudinal du tenon 6, et une face circulaire avant 17b, de rayon R3, plus grand que le rayon R2 de la face équivalente 17a de l'excroissance 15.

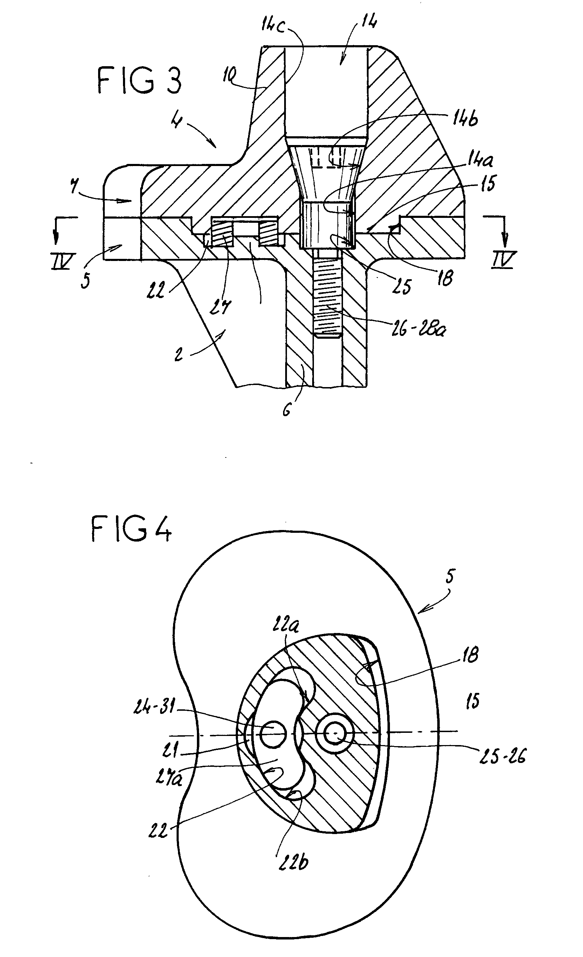

Dans la partie arrière de l'excroissance 15, est ménagé un logement 20 en forme de haricot. Les bords, respectivement antérieur 20a et postérieur 20b du logement 20 sont en forme d'arc de cercle, de rayons respectivement R4 et R5, centrés sur l'axe de l'alésage 14, tandis que les extrémités arrondies 20c sont disposées symétriquement par rapport au plan médian transversal de la pièce 4. Enfin, comme montré à la figure 3, l'alésage 14 de l'insert 4 est composé en allant du bas vers le haut d'une partie cylindrique de guidage 14a, d'une partie conique de blocage 14b et d'une partie cylindrique de dégagement 14c, cette dernière partie étant de plus grand diamètre que celui de la partie 14a.

Comme montré à la figure 2, du fond 19 du logement 18 de l'embase 2, débouche un logement 22, également en forme de haricot, et dont les faces, respectivement antérieure 22a et 22b, sont en forme d'arc de cercle, de rayons centrés sur l'axe longitudinal de l'embase tibiale et de même valeur que les rayons R4 et R5 du logement 20 de l'insert 4. Ce logement 22 est également symétrique par rapport au plan médian longitudinal et antéro-postérieur de l'embase tibiale, mais s'étend sur un arc de cercle plus grand, comme montré à la figure 4. Du fond 23 du logement 22, fait saillie un doigt vertical 24 dont l'axe longitudinal est dans le plan médian transversal de l'embase.

Enfin, du fond 19 du logement 18 de l'embase 2, débouche un alésage cylindrique épaulé 25 précédant un alésage fileté et axial 26, réalisé dans le tenon 6.

Cette prothèse comprend également trois autres éléments, à savoir une cale 27 utilisable avec une vis 28, et un pivot 29.

La cale 27 est en forme de haricot et présente des forme et dimensions lui permettant de s'engager sans jeu, pour partie dans le logement 20 de l'excroissance 15 de l'insert 4 et, pour partie, dans le logement 22 de l'embase tibiale 2. Cette cale est traversée par un alésage 31, apte à coiffer le pion 24 saillant du fond du logement 22 de l'embase 2.

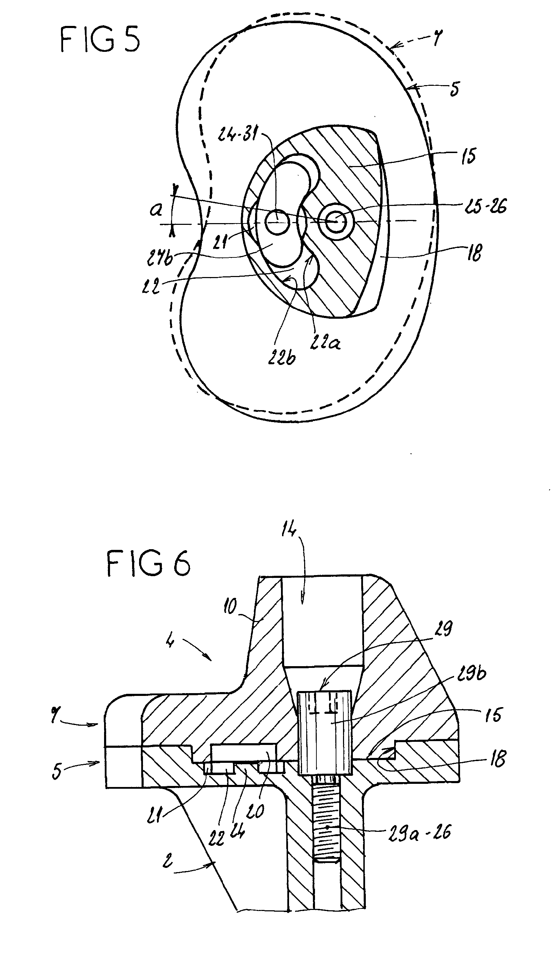

La cale fait partie d'une série de plusieurs cales se différenciant par la position de l'axe de leur alésage 31 par rapport au plan médian transversal de la cale, comme montré pour la cale 27b de figure 5, ou décalé angulairement de ce plan médian d'angle a.

La vis 28 est composée d'une partie filetée 28a, apte à se visser dans l'alésage fileté 26 de l'embase, d'une partie cylindrique 28b apte à pénétrer dans l'alésage 14a de l'insert 4, et d'une tête tronconique 28c apte à venir en appui contre le cône 14b de l'insert. Quant au pivot 29, il est composé d'une partie filetée 29a et d'une partie cylindrique 29b.

Avec ces différents éléments, la prothèse peut prendre diverses configurations s'adaptant à la pathologie et à la morphologie du patient. Avec une cale 27 symétrique et une vis 28, elle constitue, comme montré aux figures 3 et 4, une prothèse standard postéro-stabilisée de première intention dans laquelle l'insert 4 est calé en rotation par rapport à l'embase tibiale 2 par la cale 27. En effet, dans cette configuration, et comme montré figures 3 et 4, la cale est liée à l'embase par le doigt 24 et bloque en rotation l'insert par ses extrémités arrondies qui sont en butée sur celles 20c du logement 20 de cet insert.

La figure 5 montre que la même prothèse peut être adaptée à la morphologie du patient par remplacement de la cale symétrique 27 par une cale asymétrique 27b pour donner, à l'insert 4b, représentée en traits mixtes, une position angulaire différente par rapport à celle du plateau 9 de l'embase tibiale. Selon le décalage angulaire de l'axe de l'alésage 31 par rapport au plan médian de la cale 27, c'est-à-dire selon la cale choisie parmi une série de cales se différenciant par le décalage angulaire de l'ordre de 2°, mais aussi par retournement, le chirurgien peut, sans difficulté, donner à l'insert un positionnement angulaire parfaitement adapté à la morphologie du patient et sans qu'il soit nécessaire de modifier les éléments principaux et les plus coûteux de la prothèse.

Lorsque la cale est mise en place, la vis 28 est engagée dans l'alésage 14 de l'insert 4 et est visée jusqu'à ce que son cône 28c rencontre le cône 14b et assure le calage en translation transversale de l'insert 4 par rapport à l'embase tibiale 2.

Les figures 4 et 5 mettent en évidence que, grâce à sa forme s'étendant sur un plus grand arc de cercle, le logement 22 ménagé dans l'embase 2 ne gêne en rien les modifications de positionnement procurées par les cales asymétriques 27b.

Pour faciliter le réglage de ce calage angulaire en remplaçant une cale asymétrique par une autre, décalée différemment, le logement 22 comporte dans ses faces 22a, 22b, des encoches de préhension 21.

Lorsqu'il n'est pas nécessaire de bloquer l'insert 4 par rapport à l'embase tibiale 2, et que, en conséquence, cet insert peut être mobile en rotation par rapport au plateau tibial 5, la cale 27 n'est pas mise en place et la vis 28 est remplacée par le pivot 29, comme montré à la figure 6. Dans ces conditions, le pivotement de l'insert 4 par rapport au plateau 5 de l'embase tibiale 2 est limité par butée des extrémités des faces 17a et 17b, respectivement de l'excroissance 15 et du logement 18, comme le laisse entrevoir la figure 5 concernant une autre configuration.

Les figures 3 et 6 montrent que l'alésage épaulé 25 assure le positionnement radial et longitudinal de la vis 28 et du pivot 29 en coopérant avec leur partie cylindrique épaulée, respectivement 14a et 29b.

La figure 7 montre que, moyennant l'adjonction aux composants déjà décrits de la prothèse, respectivement, de rallonges fémorales 30 se vissant dans un puits fileté ménagé à l'extrémité du tenon 6 de l'embase tibiale, de rallonges fémorales 32 se vissant dans un puits fileté 33 ménagé dans un éperon 34 de l'élément condylien 3, et des cales de rattrapage 35 s'insérant sous le plateau 5 de l'embase tibiale, il est possible de donner à la prothèse la configuration d'une prothèse de reprise pouvant, elle-même, prendre l'une ou l'autre des configurations précédemment décrites avec adjonction de cales symétriques 27a ou asymétriques 27b.

Il ressort de ce qui précède que la prothèse, selon l'invention, permet non seulement au chirurgien de choisir la version qui lui convient en fonction de la morphologie et de la pathologie du patient, tout en étant d'assemblage simple et en nécessitant la même instrumentation et le même ancillaire, mais qu'elle apporte aussi des avantages économiques, puisque, en utilisant les mêmes composants principaux et les plus onéreux dans toutes les versions, elle permet d'abaisser le coût de fabrication de ces composants et de réduire les frais de stockage et de gestion.

## Revendications

1. Prothèse de genou, totale et modulaire, comprenant :
- une embase tibiale (2) avec un plateau (5) solidaire d'un tenon axial (6) enfichable dans la cavité médullaire tibiale,
- un élément condylien (3) avec un tenon axial (34) enfichable dans la cavité médullaire fémorale,
- un insert intermédiaire (4) avec un plateau (7) venant en appui sur le plateau tibial (5) et présentant, de l'autre côté, des surfaces (13) de roulement pour les condyles (12) de l'élément condylien, et un éperon (10) saillant vers le haut, cet éperon et le plateau (7) étant traversés par un alésage axial (14), coaxial à l'axe longitudinal du tenon (6) de l'élément tibial,
- et des moyens complémentaires ménagés sur le plateau tibial (5) et l'insert pour limiter leur rotation relative autour de l'axe longitudinal précité,
**caractérisée en ce que** de la face inférieure du plateau (7) de l'insert (4) débouche un logement (20) en forme de haricot dont les rayons (R4, R5) des bords en arc de cercle, respectivement antérieur et postérieur, sont centrés sur l'axe longitudinal de l'alésage axial (14) et dont les arrondis extrêmes (20c) sont disposés symétriquement par rapport au plan antéro-postérieur de l'insert, tandis que le plateau tibial (5) comporte, en vis à vis du logement (20) de l'insert, un autre logement (22) en forme de haricot, de même largeur mais s'étendant symétriquement sur un plus grand arc, ce logement (22) comportant, en saillie de son fond, un doigt (24) cylindrique et vertical, et que, dans les configurations de la prothèse où l'insert (4) est calé en rotation par rapport au plateau tibial (5), la position angulaire de l'insert (4) par rapport à ce plateau tibial est définie par une cale (27a, 27b) en forme de haricot, de même forme et dimensions que celles du logement (20) de l'insert (4), insérée pour partie dans ce logement (20) et pour partie dans celui (22) du plateau tibial (5), ladite cale étant munie d'un alésage (31) coiffant le doigt (24) saillant du fond du logement (20) du plateau tibial et assurant son calage par rapport à ce plateau et étant choisie dans une série de plusieurs cales (27, 27b) se différenciant par la position de l'axe de leur alésage (31) par rapport à leur plan médian transversal.

2. Prothèse de genou selon la revendication 1, **caractérisée en ce que** le logement (20) en forme de haricot de l'insert (4) débouche d'une excroissance (15) saillant de la face d'appui (8) de cet insert, cette excroissance étant délimitée, vers l'arrière, par une face circulaire (16a) dont le rayon (R1) est centré sur l'axe longitudinal de l'alésage (14) de l'insert et, vers l'avant, par une face circulaire (17a) de grand rayon (R4), cette excroissance (15) étant engagée dans un logement (18) ménagé dans le plateau tibial (5) et délimitée vers l'arrière, par une face circulaire (16b) de même rayon (R1) centré sur l'axe longitudinal du tenon tibial (6), et vers l'avant par une face circulaire (17b) de plus grand rayon (R3) que celui de la face (17a) correspondante de l'excroissance (15), de manière que, en configuration sans cale, donc avec rotation libre de l'insert (4), les extrémités des deux faces circulaires (17a, 17b) de grands rayons (R3, R4) constituent butées de limitation de course angulaire.

3. Prothèse de genou selon la revendication 1, **caractérisée en ce que** l'alésage axial de l'éperon (10) de l'insert est composé, en allant du bas vers le haut, d'une partie cylindrique (14a) de guidage, d'un cône (14b) de blocage et d'une partie cylindrique (14c) de dégagement, ayant un plus grand diamètre que celui de la partie de guidage (14a), tandis que du plateau tibial (5) débouche un alésage fileté (26) recevant, en configuration sans cale, donc avec rotation libre, un pivot (29) dont la partie cylindrique (29b) se loge dans la partie cylindrique de guidage (14a) de l'insert, et, en configuration avec cale (27), donc avec rotation bloquée, une vis de blocage (28) munie d'une portée extrême tronconique (28c) venant en appui sur le cône de blocage (14b) de l'insert (4).

4. prothèse de genou selon la revendication 3, **caractérisée en ce que** l'alésage fileté (26) du tenon tibial (6) débouche dans un alésage cylindrique épaulé (25) de positionnement du pivot (29) et de la vis de blocage (28).
